# FASCICULE DE BREVET EUROPEEN

(11) **EP 4 203 840 B1**
(45) Date de publication et mention de la délivrance du brevet: **09.07.2025**
(21) Numéro de dépôt: 21798754.4
(22) Date de dépôt: 05.10.2021
(51) Int. Cl.: A61B 90/00, A61B 34/30, A61B 34/10, A61B 34/20, A61B 90/50, A61B 17/00

(54) **SYSTÈME DE NAVIGATION EN RÉALITÉ AUGMENTÉE POUR UN ROBOT MÉDICAL**
NAVIGATIONSSYSTEM MIT ERWEITERTER REALITÄT FÜR EINEN MEDIZINISCHEN ROBOTER
AUGMENTED-REALITY NAVIGATION SYSTEM FOR A MEDICAL ROBOT

(30) Priorité: 08.10.2020 FR 2010309
(43) Date de publication de la demande: 05.07.2023
(73) Titulaire: Quantum Surgical, 34000 Montpellier (FR)
(72) Inventeur: BLONDEL, Lucien, 34070 Montpellier (FR); NAHUM, Bertin, 34170 Castelnau-le-Lez (FR); BADANO, Fernand, 69006 Lyon (FR)
(74) Mandataire: Ipside
(86) Numéro de dépôt international: PCT/FR2021/051719
(87) Numéro de publication internationale: WO 2022/074325

(56) Documents cités:
- WO-A1-2018/032083
- WO-A1-2019/051464
- WO-A1-2020/096810
- US-A1- 2009 156 951

## Description

### Domaine de l'invention

La présente invention appartient au domaine des équipements utilisés dans le cadre d'interventions chirurgicales mini-invasives assistées par un dispositif robotisé. Notamment, l'invention concerne un système de navigation en réalité augmenté destiné à coopérer avec un robot médical pour assister un praticien lors d'une intervention chirurgicale.

### Etat de la technique

Les interventions chirurgicales mini-invasives consistent à insérer un ou plusieurs instruments médicaux dans une anatomie d'intérêt d'un patient. Par exemple, une intervention chirurgicale mini-invasive peut viser à réaliser une biopsie ou une ablation d'une lésion telle qu'une tumeur.

Dans une intervention chirurgicale mini-invasive, l'anatomie d'intérêt du patient n'est généralement pas visible à l'œil nu par le praticien. L'insertion d'un instrument médical est généralement guidée par imagerie médicale. En vue d'améliorer la précision de l'insertion de l'instrument médical, les interventions mini-invasives peuvent être assistées par un dispositif robotisé. Afin de rendre l'anatomie d'intérêt visible pour le praticien, il est possible de transformer le contenu d'une image médicale de l'anatomie d'intérêt en un contenu de réalité augmenté superposé au corps du patient. Pour suivre l'insertion de l'instrument médical dans l'anatomie d'intérêt pendant l'intervention chirurgicale, il convient cependant d'obtenir de nombreuses images médicales, ce qui nécessite d'utiliser des moyens invasifs (par exemple un endoscope) ou bien des dispositifs d'imagerie médicale qui exposent le patient à des irradiations tout au long de l'intervention chirurgicale.

Dans le cas particulier des organes mous, les images médicales ne peuvent pas reproduire les mouvements liés à la respiration, aux déformations locales de l'organe dues à l'insertion de l'instrument médical ou encore aux déplacements involontaires du patient au moment de l'intervention. Ainsi, la position d'une lésion dans une anatomie d'intérêt peut être différente lors de l'acquisition d'une image médicale et lors de l'intervention. Si l'insertion de l'instrument médical est planifiée à partir de l'image médicale, il y a un risque que la lésion ne soit pas atteinte avec précision par l'instrument médical.

Les moyens actuels pour assister un praticien lors d'une intervention chirurgicale mini-invasive ne permettent donc pas au praticien de prendre en compte de manière simple et fiable les mouvements liés à la respiration du patient, à des déformations internes de l'anatomie d'intérêt, ou à des déplacements involontaires du patient au cours de l'intervention chirurgicale.

Le document WO 2019/051464 décrit un système de navigation en réalité augmentée pour assister un praticien lors d'une intervention chirurgicale sur une anatomie d'intérêt d'un patient, ledit système comprenant:
- une caméra pour acquérir des images réelles, ladite caméra étant destinée à être portée par le praticien au niveau de la tête,
- un dispositif d'affichage pour afficher en temps réel les images réelles acquises par la caméra ainsi qu'un contenu de réalité augmentée superposé auxdites images réelles,
- une unité de contrôle connectée à la caméra et au dispositif d'affichage, l'unité de contrôle étant configurée pour:
- détecter sur les images réelles la position d'un marqueur placé sur le patient à proximité de ladite anatomie d'intérêt,
- enregistrer le mouvement suivi par le marqueur pendant une période d'enregistrement correspondant à un ou plusieurs cycles respiratoires du patient, et
- déterminer la position du marqueur à un premier instant appartenant à ladite période d'enregistrement et correspondant à un instant d'acquisition d'une image médicale pré-interventionnelle de l'anatomie d'intérêt du patient par un dispositif d'imagerie médicale.

### Exposé de l'invention

La présente invention a pour objectif de remédier à tout ou partie des inconvénients de l'art antérieur, notamment ceux exposés ci-avant.

A cet effet, et selon un premier aspect, il est proposé par la présente invention un système de navigation en réalité augmentée pour assister un praticien lors d'une intervention chirurgicale sur une anatomie d'intérêt d'un patient. Ledit système comprend notamment :
- une caméra pour acquérir des images réelles, ladite caméra étant destinée à être portée par le praticien au niveau de la tête,
- un dispositif d'affichage pour afficher en temps réel les images réelles acquises par la caméra ainsi qu'un contenu de réalité augmentée superposé auxdites images réelles,
- une unité de contrôle connectée à la caméra et au dispositif d'affichage.

Ladite unité de contrôle du système de navigation en réalité augmentée est configurée pour:
- détecter sur les images réelles la position d'un marqueur placé sur le patient à proximité de ladite anatomie d'intérêt,
- enregistrer le mouvement suivi par le marqueur pendant une période d'enregistrement correspondant à un ou plusieurs cycles respiratoires du patient,
- recevoir une information qu'une image médicale pré-interventionnelle de l'anatomie d'intérêt du patient a été acquise par un dispositif d'imagerie médicale à un premier instant appartenant à ladite période d'enregistrement, et déterminer la position et l'orientation du marqueur audit premier instant,
- élaborer un modèle prédictif du mouvement du marqueur à partir du mouvement suivi par le marqueur pendant la période d'enregistrement.

Dans la présente demande, et sauf indication contraire, le terme « position » englobe à la fois la notion d'emplacement et d'orientation d'un objet dans un repère donné qui est généralement un système de coordonnées en trois dimensions. Le terme « pose » est employé dans la littérature anglo-saxonne pour représenter cette combinaison de la position et de l'orientation d'un objet dans l'espace. Dans la présente demande, et sauf indication contraire, le terme « position » est donc équivalent au terme « position/orientation ».

L'intervention chirurgicale mini-invasive consiste par exemple à insérer dans une anatomie d'intérêt d'un patient (par exemple un organe mou tel que le foie, un poumon, un rein ou une structure rigide telle qu'un os) un ou plusieurs instruments médicaux pour réaliser une biopsie, pour ablater une lésion (par exemple une tumeur), pour positionner un implant (par exemple une vis, une plaque, une prothèse), ou pour insérer d'autres matériaux (par exemple ciment ou disque artificiel). Dans ce type d'intervention, l'anatomie d'intérêt n'est pas visible à l'œil nu.

Avantageusement, la caméra du système de navigation en réalité augmentée est portée par le praticien au niveau de la tête pour maintenir une ligne de vue directe du patient pour la caméra (notamment, le praticien ne peut pas faire obstacle à cette ligne de vue directe).

Le système de navigation en réalité augmentée permet d'afficher des informations sur le dispositif d'affichage sous forme de contenu de réalité augmentée, c'est-à-dire que ces informations sont superposées aux images réelles, et elles ne seraient autrement pas visibles par le praticien. De manière avantageuse, le dispositif d'affichage peut également être porté par le praticien au niveau de la tête, directement devant les yeux du praticien (par exemple sous la forme d'un masque, d'un casque ou de lunettes). De telles dispositions permettent au praticien de lire des informations sur le dispositif d'affichage sans détourner son regard du patient. Cela permet également de limiter l'encombrement de la salle d'intervention. Rien n'empêche toutefois que, dans des variantes, le dispositif d'affichage soit un écran visible par plusieurs personnes présentes dans la salle d'intervention (par exemple un écran fixé à la table d'intervention au-dessus du patient).

L'unité de contrôle peut également être portée par le praticien au niveau de la tête. Alternativement, l'unité de contrôle peut appartenir à une entité distincte de l'entité portée par le praticien au niveau de la tête. L'unité de contrôle est connectée à la caméra et au dispositif d'affichage, par exemple via des moyens de communication filaire ou via des moyens de communication sans fil.

Le marqueur placé sur le patient à proximité de l'anatomie d'intérêt peut être repéré par le système de navigation en réalité augmentée grâce à un ou plusieurs marqueurs optiques dont la géométrie est connue. Ce marqueur comprend également des marqueurs radio opaques dont la géométrie est connue et qui sont visibles sur une image médicale acquise par un dispositif d'imagerie médicale (par exemple par tomodensitométrie, par résonance magnétique, par ultrasons, par tomographie, par émission de positions, etc.). L'image médicale pré-interventionnelle est par exemple acquise à un instant où la respiration du patient est bloquée. Rien n'empêche cependant, selon un autre exemple, que l'image médicale pré-interventionnelle soit acquise à un instant où le patient respire librement, par exemple lorsque la respiration atteint un plateau en fin d'inspiration ou en fin d'expiration (lorsque l'un de ces plateaux est atteint, le mouvement lié à la respiration du patient est négligeable pendant deux à trois secondes).

Il est possible de planifier l'intervention chirurgicale à partir de l'image médicale pré-interventionnelle, et notamment de définir la position que doit prendre l'instrument médical par rapport à la position du marqueur pour effectuer ladite intervention chirurgicale. Toutefois, la position de l'anatomie d'intérêt peut varier par rapport à la position du marqueur en raison des mouvements liés à la respiration du patient, aux déformations internes de l'organe ou aux déplacements involontaires du patient au moment de l'intervention. Il convient donc de s'assurer que lorsque l'intervention chirurgicale est exécutée, le patient se trouve dans la même position ou dans la même phase du cycle respiratoire qu'au moment où l'image médicale pré-interventionnelle a été acquise.

Dans l'invention, l'unité de contrôle est configurée pour enregistrer le mouvement suivi par le marqueur pendant un ou plusieurs cycles respiratoires du patient, pour déterminer la position du marqueur à l'instant où l'image médicale pré-interventionnelle est acquise, et pour élaborer un modèle prédictif du mouvement du marqueur à partir du mouvement enregistré.

De telles dispositions permettent notamment de planifier l'intervention chirurgicale sur l'image médicale pré-interventionnelle et de s'assurer qu'au moment de l'insertion de l'instrument médical, le marqueur se trouve à la même position que la position du marqueur au moment où l'image médicale pré-interventionnelle a été acquise (ou autrement dit de s'assurer qu'au moment de l'insertion de l'instrument médical, le patient se trouve dans la même position ou dans la même phase du cycle respiratoire qu'au moment où l'image médicale pré-interventionnelle a été acquise).

D'autre part, le modèle prédictif du mouvement du marqueur peut être utilisé pour mettre à jour de façon continue et en temps réel un modèle anatomique en trois dimensions de l'anatomie d'intérêt généré à partir de l'image médicale pré-interventionnelle. Ce modèle anatomique peut alors être affiché sur le dispositif d'affichage sous forme d'un contenu de réalité augmenté superposé aux images réelles.

Dans sa forme la plus simple, le modèle prédictif correspond au mouvement du marqueur enregistré pendant un cycle respiratoire, ou pendant plusieurs cycles respiratoires. De manière avantageuse, le modèle prédictif peut toutefois être élaboré à l'aide de calculs statistiques sur les positions prises par le marqueur pendant plusieurs cycles respiratoires consécutifs. Il est notamment envisageable de recourir à des algorithmes d'apprentissage automatique pour élaborer le modèle prédictif.

Le dispositif d'imagerie médicale est une entité distincte du système de navigation en réalité augmentée. Différents moyens de synchronisation sont envisageables pour que le système de navigation en réalité augmentée détecte l'instant auquel l'image médicale pré-interventionnelle est acquise.

Le système de navigation en réalité augmentée détecte la position du marqueur sur les images réelles. Cette détection de la position du marqueur peut éventuellement être facilitée ou fiabilisée par un dispositif de navigation optique (caméra stéréoscopique, caméra temps de vol, etc.) ou un dispositif de navigation électromagnétique.

Dans des modes particuliers de réalisation, l'invention peut comporter en outre l'une ou plusieurs des caractéristiques suivantes, prises isolément ou selon toutes les combinaisons techniquement possibles.

Dans des modes particuliers de réalisation, l'unité de contrôle est en outre configurée pour :
- déterminer à partir du modèle prédictif un deuxième instant correspondant à un instant candidat d'insertion d'un instrument médical dans l'anatomie d'intérêt du patient,
- comparer la position du marqueur au premier instant avec la position du marqueur au deuxième instant,
- afficher un résultat de la comparaison sur le dispositif d'affichage sous forme d'un contenu de réalité augmenté superposé aux images réelles.

La comparaison de la position du marqueur au premier instant (qui correspond à l'instant d'acquisition de l'image médicale pré-interventionnelle pendant la période d'enregistrement) avec la position du marqueur au deuxième instant (qui correspond à l'insertion de l'instrument médical dans l'anatomie d'intérêt) permet alors de vérifier si le marqueur se trouve à la même position que la position du marqueur au moment où l'image médicale pré-interventionnelle a été acquise (ou autrement dit de vérifier si au moment de l'insertion de l'instrument médical, le patient se trouve dans la même phase du cycle respiratoire qu'au moment où l'image médicale pré-interventionnelle a été acquise). Le deuxième instant correspond par exemple à un instant où la respiration du patient est bloquée. Rien n'empêche toutefois que le patient respire librement pendant l'intervention. Le deuxième instant peut être déterminé à l'aide du modèle prédictif en synchronisant le mouvement du marqueur dû à la respiration du patient avec le modèle prédictif.

Dans des modes particuliers de réalisation, l'unité de contrôle est en outre configurée pour recevoir l'image médicale pré-interventionnelle du dispositif d'imagerie médicale et pour générer à partir de l'image médicale pré-interventionnelle un modèle anatomique en trois dimensions de l'anatomie d'intérêt du patient et pour afficher ledit modèle anatomique sur le dispositif d'affichage sous forme d'un contenu de réalité augmenté superposé aux images réelles. La position du modèle anatomique superposé aux images réelles est mise à jour de façon continue et en temps réel en fonction du modèle prédictif du mouvement du marqueur et en fonction d'un modèle biomécanique de structures anatomiques du corps humain

Par « modèle biomécanique », on entend un modèle mathématique des différentes structures anatomiques (muscles, tendons, structures osseuses, organes, réseau vasculaire, etc.) du corps humain et donc du patient dans la zone anatomique considérée qui permet de modéliser les déformations desdites structures anatomiques ainsi que les interactions mécaniques entre lesdites structures anatomiques. Un tel modèle biomécanique permet donc, notamment, de déterminer les déformations et les interactions mécaniques (et donc les déplacements) des structures anatomiques internes du patient induites, par exemple, par une modification de l'enveloppe externe dudit patient, une modification des positions des vaisseaux d'un organe, une modification de l'enveloppe externe d'un organe, etc. De telles modifications peuvent par exemple être induites par la respiration du patient (déplacement des organes induit par le mouvement de la cage thoracique et du diaphragme), par un changement de position du patient (déplacement des organes induit par gravité), par un contact avec un instrument médical (déformation locale), etc.

Le mouvement du marqueur est par exemple représentatif du mouvement de la cage thoracique du patient provoqué par la respiration du patient. Le modèle biomécanique permet alors de définir comment la position du modèle anatomique est impactée par ces mouvements au cours des cycles respiratoires du patient.

Avec de telles dispositions, le praticien peut visualiser sur le dispositif d'affichage l'anatomie d'intérêt « à travers » l'enveloppe externe du corps du patient, alors que l'anatomie d'intérêt n'est pas visible à l'œil nu puisqu'elle est interne au corps du patient.

Dans des modes particuliers de réalisation, l'unité de contrôle est en outre configurée pour afficher sur le modèle anatomique, sous forme d'un contenu de réalité augmenté superposé aux images réelles, une trajectoire destinée à être suivie par un instrument médical, ladite trajectoire étant mise à jour en temps réel en fonction de la position du modèle anatomique.

La trajectoire qui doit être suivie par l'instrument médical est une donnée de planification de l'intervention chirurgicale déterminée par exemple à partir de l'image médicale pré-interventionnelle. Cette trajectoire définit notamment la position que doit prendre l'instrument médical par rapport à la position du marqueur pour effectuer ladite intervention chirurgicale. Cette trajectoire comporte par exemple un point cible à atteindre dans une région à traiter (par exemple une tumeur) au sein de l'anatomie d'intérêt, et un point d'entrée de l'instrument médical à la surface de l'anatomie d'intérêt.

Dans des modes particuliers de réalisation, la trajectoire destinée à être suivie par l'instrument médical est définie a priori sur une image médicale pré-opératoire, et l'unité de contrôle est configurée pour recevoir ladite image médicale pré-opératoire et pour effectuer un recalage de l'image pré-opératoire avec l'image médicale pré-interventionnelle pour afficher la trajectoire sur le modèle anatomique.

On entend par image « pré-opératoire » une image sur laquelle une pathologie est diagnostiquée plusieurs jours, plusieurs semaines, voire plusieurs mois avant une intervention chirurgicale pour traiter ladite pathologie. On entend par image « pré-interventionnelle » une image acquise au moment de l'intervention chirurgicale lorsque le patient est installé sur la table d'intervention mais avant la réalisation du geste chirurgical (c'est-à-dire avant l'insertion de l'instrument médical).

Dans des modes particuliers de réalisation, le système comporte en outre un dispositif d'interaction permettant au praticien de pointer un endroit particulier sur le modèle anatomique et dans lequel la trajectoire destinée à être suivie par l'instrument médical est définie par le praticien à l'aide du dispositif d'interaction.

Dans des modes particuliers de réalisation, l'unité de contrôle est configurée pour segmenter au moins un élément sur le modèle anatomique parmi les éléments suivants:
- différentes structures anatomiques au sein de l'anatomie d'intérêt,
- une région à traiter au sein de l'anatomie d'intérêt,
- une région d'ablation estimée à partir de paramètres de l'intervention chirurgicale,
- des marges d'ablation d'une région à traiter au sein de l'anatomie d'intérêt déterminées en comparant la région à traiter et la région d'ablation estimée,
et pour afficher ledit élément segmenté sur le modèle anatomique sous forme d'un contenu de réalité augmenté superposé aux images réelles. L'élément segmenté est alors mis à jour en temps réel en fonction de la position du modèle anatomique.

Par « structures anatomiques », on entend par exemple des organes, des os, des vaisseaux sanguins, etc. Une région à traiter correspond par exemple à une tumeur à ablater. Une région d'ablation peut être estimée en fonction des paramètres de l'intervention chirurgicale, comme par exemple un type de traitement, un type d'instrument médical utilisé pour le traitement, une durée du traitement, une puissance du traitement, etc.

Dans des modes particuliers de réalisation, l'unité de contrôle est configurée pour afficher sur le dispositif d'affichage, sous forme d'un contenu de réalité augmenté, au moins un objet virtuel de configuration concernant un paramètre de traitement chirurgical. Le système comporte un dispositif d'interaction permettant au praticien d'interagir avec ledit objet virtuel de configuration afin de sélectionner une valeur particulière pour ledit paramètre.

Un objet virtuel de configuration correspond par exemple à un menu, un bouton, une liste de choix multiples, etc., permettant de définir une valeur particulière de paramètre pour le traitement à effectuer.

Dans des modes particuliers de réalisation, le dispositif d'affichage est destiné à être porté par le praticien au niveau des yeux du praticien.

Dans des modes particuliers de réalisation, l'unité de contrôle est configurée pour modifier la position d'un contenu de réalité augmentée en fonction de la position de la tête du praticien.

Dans des modes particuliers de réalisation, la position de la tête du praticien est déterminée à l'aide d'un capteur de mouvement de la tête du praticien ou directement à partir de la position du marqueur sur les images réelles

Selon un deuxième aspect, la présente invention concerne un ensemble formé par un système de navigation en réalité augmentée selon l'un quelconque des modes de réalisation précédents et un robot médical pour assister un praticien lors d'une intervention chirurgicale sur une anatomie d'intérêt d'un patient. Le robot médical comporte une base mobile, un bras articulé, et une unité de contrôle. Une extrémité du bras articulé est fixée à la base mobile et l'autre extrémité présente un guide outil destiné à maintenir un instrument médical. L'unité de contrôle du robot médical est configurée pour déterminer une configuration du bras articulé qui permette d'effectuer un geste chirurgical avec l'instrument médical selon une trajectoire prédéterminée. La configuration du bras articulé est déterminée en fonction d'une information relative à la position du marqueur transmise par le système de navigation.

Le système de navigation en réalité augmentée et le robot médical coopèrent l'un avec l'autre. Le système de navigation en réalité augmentée et le robot médical comportent par exemple des moyens de communication pour échanger des messages. Ces moyens de communication peuvent être de type filaire ou sans fil.

Le système de navigation en réalité augmentée transmet au robot médical une information relative à la position du marqueur pour que le robot médical positionne son bras articulé dans une configuration qui permet d'effectuer un geste chirurgical avec l'instrument médical selon une trajectoire prédéterminée. La trajectoire qui doit être suivie par l'instrument médical est une donnée de planification de l'intervention chirurgicale déterminée par exemple à partir de l'image médicale pré-interventionnelle.

Le geste médical peut être effectué par le praticien (dans ce cas le bras articulé du robot médical sert principalement à guider l'instrument médical pour assister le praticien dans son exécution du geste médical) ou directement par le robot médical.

Dans des modes particuliers de réalisation, l'invention peut comporter en outre l'une ou plusieurs des caractéristiques suivantes, prises isolément ou selon toutes les combinaisons techniquement possibles.

Dans des modes particuliers de réalisation, l'information relative à la position du marqueur est une indication que le résultat de la comparaison de la position du marqueur au premier instant (qui correspond à l'instant d'acquisition de l'image médicale pré-interventionnelle pendant la période d'enregistrement) avec la position du marqueur au deuxième instant (qui correspond à un instant candidat pour l'insertion de l'instrument médical dans l'anatomie d'intérêt) satisfait un critère particulier.

Par exemple, la respiration du patient peut être bloquée pour insérer l'instrument médical. Au moment où la respiration est bloquée, si la position du marqueur est suffisamment proche de la position du marqueur à l'instant où l'image médicale pré-interventionnelle a été acquise, alors le système de navigation en réalité augmentée transmet une indication au robot médical que l'intervention peut avoir lieu telle qu'elle a été planifiée à partir de l'image médicale pré-interventionnelle (car le patient se trouve dans la même phase du cycle respiratoire qu'au moment où l'image médicale pré-interventionnelle a été acquise).

L'indication peut être transmise par le système de navigation en réalité augmentée après validation du résultat de la comparaison par le praticien, ou bien de façon automatique par exemple si la différence de position est inférieure à un seuil prédéterminé.

Dans des modes particuliers de réalisation, l'information relative à la position du marqueur correspond à un modèle prédictif représentatif de la position du modèle anatomique au cours des cycles respiratoires du patient. L'unité de contrôle du robot médical est configurée pour ajuster de façon continue et en temps réel la configuration du bras articulé en fonction dudit modèle prédictif représentatif de la position du modèle anatomique.

Avec de telles dispositions, le bras articulé est positionné dans une configuration qui permet d'effectuer l'intervention chirurgicale quel que soit l'instant considéré dans un cycle respiratoire du patient.

Dans des modes particuliers de réalisation, l'unité de contrôle du système de navigation en réalité augmentée est configurée pour modéliser une enveloppe externe du corps du patient, pour déterminer la position du bras articulé ou du guide outil du robot médical, et pour détecter une situation de risque de collision lorsque la distance entre le guide outil et l'enveloppe externe du patient est inférieure à un seuil prédéterminé.

L'enveloppe externe du corps du patient est par exemple modélisée à partir d'une détection des contours du corps du patient sur les images réelles. De telles dispositions peuvent permettre de mesurer de façon continue et en temps réel la distance entre le bras articulé et le patient ou la distance entre le guide outil et le patient. Des mesures immédiates peuvent alors être prises pour éviter un contact indésiré entre le bras articulé ou le guide outil et le patient lors du positionnement du bras articulé (par exemple lorsque la corpulence du patient est plus importante que ce qui avait été estimé lors de la planification de l'intervention).

Dans des modes particuliers de réalisation, l'unité de contrôle interrompt le mouvement du bras articulé dès que la distance entre le bras articulé ou le guide outil et l'enveloppe externe du patient est insuffisante, par exemple inférieure à 5 cm, voire inférieure à 1 cm.

Dans des modes particuliers de réalisation, l'unité de contrôle du système de navigation en réalité augmentée est configurée pour détecter une situation de risque de blessure par l'instrument médical lorsqu'une déviation de la position du marqueur par rapport au modèle prédictif du mouvement du marqueur est supérieure à un seuil prédéterminé. De telles dispositions permettent de détecter une situation dans laquelle le patient effectue un mouvement inattendu au cours de l'insertion de l'instrument médical ou lorsque l'instrument médical est inséré mais pas encore libéré du guide outil. Une telle situation peut en effet conduire à une blessure du patient par l'instrument médical (par exemple endommagement des tissus sains de l'anatomie d'intérêt ou d'une autre partie du corps du patient par l'instrument médical). Des mesures immédiates peuvent alors être prises pour éviter de blesser le patient lorsqu'une telle situation est détectée.

Dans des modes particuliers de réalisation, le guide outil du robot médical comporte un actionneur permettant de libérer instantanément l'instrument médical. L'actionneur est commandé par l'unité de contrôle du robot médical. L'unité de contrôle du système de navigation en réalité augmentée est configurée pour transmettre à l'unité de contrôle du robot médical une commande pour libérer instantanément l'instrument médical lorsque la situation de risque de blessure est détectée.

Dans des modes particuliers de réalisation, le bras articulé comporte au moins six degrés de liberté, de sorte que plusieurs configurations candidates différentes du bras articulé permettent d'effectuer le geste chirurgical avec l'instrument médical selon la trajectoire prédéterminée. L'unité de contrôle du système de navigation en réalité augmentée est configurée pour afficher sur le dispositif d'affichage, sous forme d'un contenu de réalité augmenté superposé aux images réelles, lesdites configurations candidates, et pour recevoir une indication relative à une sélection, par le praticien, d'une configuration particulière parmi les différentes configurations candidates.

Les différentes configurations candidates sont superposées aux images réelles et permettent au praticien de sélectionner une configuration particulière de telle sorte que la présence du robot médical ne le gêne pas (ou peu) pendant l'intervention chirurgicale.

Dans des modes particuliers de réalisation, lorsque l'instrument médical est inséré dans le corps du patient selon la trajectoire prédéterminée, l'unité de contrôle du système de navigation en réalité augmentée est configurée pour afficher sur le dispositif d'affichage, sous forme d'un contenu de réalité augmenté superposé aux images réelles, une partie de l'instrument médical insérée dans le corps du patient. Dans ce but, la position de l'instrument médical est par exemple détectée par l'unité de contrôle du système de navigation en réalité augmenté sur les images réelles acquises par la caméra grâce à des algorithmes connus de type « vision par ordinateur (« Computer Vision » dans la littérature anglo-saxonne).

Avec de telles dispositions, le praticien peut suivre en continu et en temps réel l'insertion de l'instrument médical dans l'anatomie d'intérêt du patient dans la mesure où la partie non visible à l'œil nu de l'instrument médical est superposée aux images réelles.

Dans des modes particuliers de réalisation, l'unité de contrôle du système de navigation en réalité augmentée est configurée pour calculer et afficher sur le dispositif d'affichage, sous forme d'un contenu de réalité augmenté superposé aux images réelles, la distance entre l'instrument médical et un point cible de l'anatomie d'intérêt et/ou pour détecter un instant où l'instrument médical a atteint ledit point cible.

### Présentation des figures

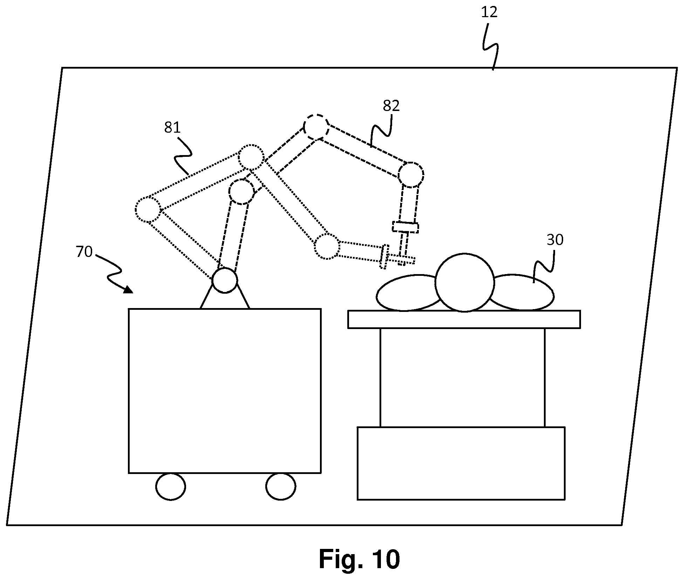

L'invention sera mieux comprise à la lecture de la description suivante, donnée à titre d'exemple nullement limitatif, et faite en se référant aux figures 1 à 10 qui représentent :
[Fig. 1] une représentation schématique d'un mode de réalisation d'un système de navigation en réalité augmentée selon l'invention,
[Fig. 2] une représentation schématique d'un marqueur destiné à être placé sur le patient à proximité de l'anatomie d'intérêt,
[Fig. 3] une représentation du mouvement suivi par le marqueur pendant une période d'enregistrement dans un plan XY du système de coordonnées du système de navigation,
[Fig. 4] une représentation en trait continu du mouvement suivi par le marqueur selon un axe principal pendant une période d'enregistrement, ainsi qu'une représentation en trait discontinu d'un modèle prédictif du mouvement du marqueur,
[Fig. 5] une illustration de la détermination, pendant la période d'enregistrement, d'un premier instant correspondant à un instant d'acquisition d'une image médicale pré-interventionnelle de l'anatomie d'intérêt du patient,
[Fig. 6] une illustration de la détermination d'un deuxième instant correspondant à un instant candidat pour l'insertion d'un instrument médical, et de la comparaison de la position du marqueur au premier instant avec la position du marqueur au deuxième instant,
[Fig. 7] une représentation schématique de la génération d'un modèle anatomique en trois dimensions de l'anatomie d'intérêt à partir d'une image médicale pré-interventionnelle,
[Fig. 8] une représentation schématique de la mise à jour en temps réel et de façon continue du modèle anatomique en fonction du modèle prédictif du mouvement du marqueur et en fonction d'un modèle biomécanique de structures anatomiques du corps humain,
[Fig. 9] une illustration de la coopération entre le système de navigation en réalité augmentée selon l'invention et un robot médical utilisé pour assister le praticien dans son intervention chirurgicale,
[Fig. 10] une représentation schématique de l'affichage, sous forme d'un contenu de réalité augmentée, de plusieurs configurations candidates du bras articulé du robot médical permettant d'effectuer l'intervention chirurgicale.

Dans ces figures, des références identiques d'une figure à une autre désignent des éléments identiques ou analogues. Pour des raisons de clarté, les éléments représentés ne sont pas nécessairement à une même échelle, sauf mention contraire.

### Description détaillée d'un mode de réalisation de l'invention

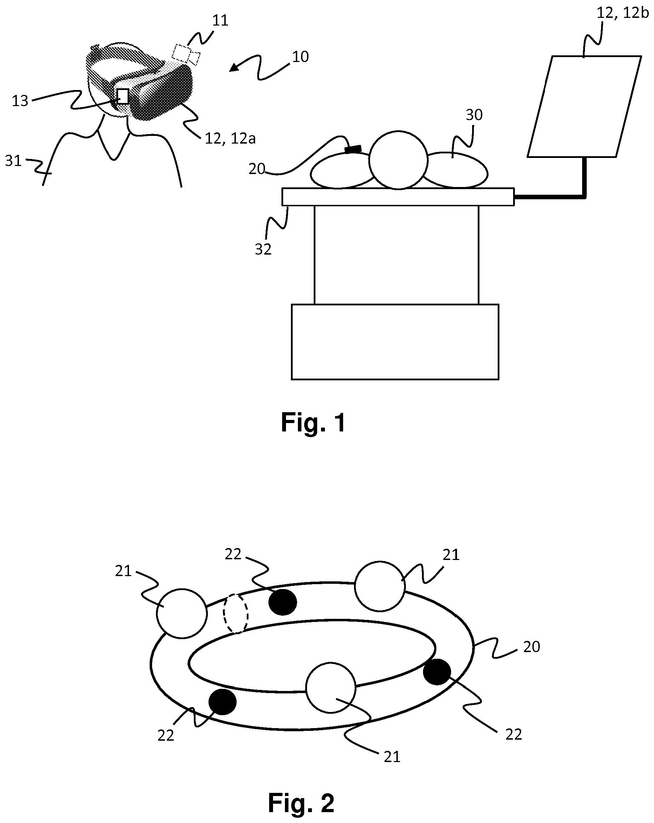

La figure 1 représente schématiquement un mode de réalisation d'un système 10 de navigation en réalité augmentée pour assister un praticien 31 lors d'une intervention chirurgicale sur une anatomie d'intérêt d'un patient 30 reposant sur une table d'intervention 32.

Le système 10 de navigation en réalité augmentée comprend une caméra 11 pour acquérir des images réelles. La caméra 11 est destinée à être portée par le praticien au niveau de la tête. Dans l'exemple considéré et illustré à la figure 1, le système 10 prend la forme d'un casque de réalité augmentée. La caméra 11 est alors intégrée au casque. De telles dispositions permettent au système 10 de navigation de maintenir une ligne de vue directe du patient 30 pour la caméra 11. Notamment, le praticien 31 ne peut pas faire obstacle à cette ligne de vue directe (comme cela pourrait être le cas si la caméra était montée sur un mât dans la salle d'intervention).

Le système 10 de navigation en réalité augmentée comprend également un dispositif d'affichage 12 pour afficher en temps réel les images réelles acquises par la caméra 11 ainsi qu'un contenu de réalité augmentée superposé auxdites images réelles. De manière avantageuse, le dispositif d'affichage 12 peut également être porté par le praticien au niveau de la tête, directement devant les yeux du praticien 31, comme c'est le cas dans l'exemple illustré à la figure 1 pour lequel le dispositif d'affichage est un écran d'affichage 12a intégré au casque de réalité augmentée. De telles dispositions permettent au praticien 31 de lire des informations sur le dispositif d'affichage sans détourner son regard du patient 30. Alternativement ou en complément, le dispositif d'affichage 12 peut également prendre la forme d'un écran d'affichage 12b, fixé par exemple à la table d'intervention 32, visible par plusieurs personnes présentes dans la salle d'intervention.

Le système 10 de navigation en réalité augmentée comprend également une unité de contrôle 13 connectée à la caméra 11 et au dispositif d'affichage 12. De manière conventionnelle, l'unité de contrôle peut être connectée à la caméra et au dispositif d'affichage 12 via des moyens de communication filaire (notamment lorsque tous ces éléments sont intégrés au casque de réalité augmentée porté par le praticien) ou via des moyens de communication sans fil (par exemple lorsque le dispositif d'affichage 12b et/ou l'unité de contrôle 13 sont déportés).

L'unité de contrôle 13 comporte un ou plusieurs processeurs pour mettre en œuvre des algorithmes de réalité augmentée qui fonctionnent sur le principe de la vision par ordinateur (« Computer Vision » dans la littérature anglo-saxonne) pour détecter des éléments distinctifs dans l'univers réel, puis y superposer du contenu virtuel. Alternativement ou en complément, l'unité de contrôle 13 comporte un ou des circuits logiques programmables (FPGA, PLD, etc.), et/ou un ou des circuits intégrés spécialisés (ASIC), et/ou un ensemble de composants électroniques discrets, etc., pour mettre en œuvre ces algorithmes. En d'autres termes, l'unité de contrôle est configurée par des moyens logiciels et/ou matériels pour mettre en œuvre ces algorithmes de réalité augmentée.

L'unité de contrôle 13 est notamment configurée pour détecter sur les images réelles la position d'un marqueur 20 placé sur le patient 30 à proximité de l'anatomie d'intérêt.

L'unité de contrôle 13 peut également être configurée pour détecter sur les images réelles la position d'un instrument médical manipulé par le praticien, ou maintenu par un guide outil fixé à l'extrémité d'un bras articulé d'un robot médical utilisé pour assister le praticien.

La figure 2 représente schématiquement un tel marqueur 20 destiné à être placé sur le patient à proximité de l'anatomie d'intérêt. Dans l'exemple considéré et illustré à la figure 2, le marqueur 20 comporte trois marqueurs optiques 21 dont la géométrie et les positions respectives sont connues. De telles dispositions permettent de déterminer avec précision la position du marqueur 20 dans un repère fixe du système 10 de navigation. Ce repère est un système de coordonnées en trois dimensions défini à partir d'un objet fixe, comme par exemple la table d'intervention 32.

Le marqueur 20 comporte également des marqueurs radio opaques 22 dont la géométrie et les positions respectives sont connues, et qui sont visibles sur une image médicale pré-interventionnelle acquise par un dispositif d'imagerie médicale (par exemple par tomodensitométrie, par résonance magnétique, par ultrasons, par tomographie, par émission de positions, etc.). Il est ainsi possible de déterminer avec précision la position d'une zone anatomique cible de l'anatomie d'intérêt par rapport à la position du marqueur 20, et de planifier l'intervention chirurgicale à partir de l'image médicale pré-interventionnelle. Il est notamment possible de définir la position que doit prendre un instrument médical par rapport à la position du marqueur 20 pour effectuer l'intervention chirurgicale.

La position de l'anatomie d'intérêt peut cependant varier par rapport à la position du marqueur 20 en raison des mouvements liés à la respiration du patient, aux déformations internes de l'organe ou aux déplacements involontaires du patient au moment de l'intervention. Il convient donc de s'assurer que lorsque l'intervention chirurgicale est exécutée, le patient se trouve dans la même position ou dans la même phase du cycle respiratoire qu'au moment où l'image médicale pré-interventionnelle a été acquise.

Dans ce but, l'unité de contrôle 13 est configurée pour enregistrer le mouvement suivi par le marqueur 20 pendant un ou plusieurs cycles respiratoires du patient 30, pour déterminer la position du marqueur 20 à l'instant où l'image médicale pré-interventionnelle est acquise (implicitement, cela signifie que l'unité de contrôle est configurée dans un premier temps pour déterminer l'instant d'acquisition de l'image médicale pré-interventionnelle, et dans un deuxième temps pour déterminer la position du marqueur audit instant d'acquisition), et pour élaborer un modèle prédictif du mouvement du marqueur à partir du mouvement enregistré. Le modèle prédictif du mouvement du marqueur permet alors de déterminer un deuxième instant auquel l'instrument médical doit être inséré dans l'anatomie d'intérêt du patient. Le deuxième instant est déterminé de telle sorte qu'au deuxième instant la position du marqueur 20 est sensiblement la même que la position du marqueur 20 au premier instant. Cela permet de s'assurer qu'au moment de l'insertion de l'instrument médical (deuxième instant), le patient se trouve dans la même position ou dans la même phase du cycle respiratoire qu'au moment où l'image médicale pré-interventionnelle a été acquise (premier instant). L'unité de contrôle 13 peut être configurée pour comparer la position du marqueur au premier instant avec la position du marqueur au deuxième instant et pour afficher un résultat de la comparaison sur le dispositif d'affichage sous forme d'un contenu de réalité augmenté superposé aux images réelles.

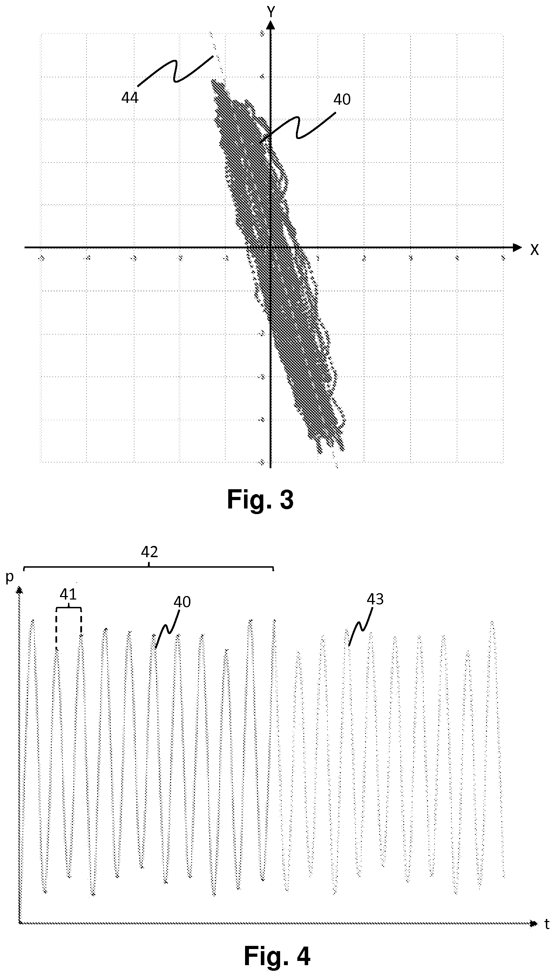

La figure 3 représente, à titre d'exemple, un enregistrement du mouvement 40 suivi par le marqueur 20 pendant une période d'enregistrement d'une durée prédéterminée correspondant à plusieurs cycles respiratoires du patient. Chaque point correspond à une position prise par le marqueur 20 au cours du temps dans un plan XY du système de coordonnées du système 10 de navigation. On constate, dans cet exemple, que le mouvement du marqueur 20 s'effectue principalement selon l'axe 44 tracé en pointillé sur la figure 3.

Le mouvement 40 du marqueur 20 est représentatif du mouvement de la cage thoracique du patient provoqué par la respiration du patient. Pour une meilleure interprétation du mouvement du marqueur, et par analogie au cycle respiratoire du patient, il est préférable d'obtenir une courbe à une dimension, illustrant le mouvement oscillatoire du marqueur dans le temps. Il existe différentes méthodes permettant d'obtenir cette courbe à une dimension. Il serait par exemple envisageable de considérer que le mouvement du marqueur est majoritairement vertical, et par conséquent de ne considérer que l'axe Y. Cependant, dans ce cas, une partie de l'amplitude du mouvement du marqueur sera perdue. Selon un autre exemple, il est envisageable d'effectuer une analyse en composantes principales des positions du marqueur. Les positions du marqueur peuvent notamment être affichées selon une composante principale correspondant à l'axe 44 principal du mouvement du marqueur.

La figure 4 représente en trait continu le mouvement 40 du marqueur 20 pendant la période d'enregistrement 42 selon l'axe 44 principal au cours du temps. La position p du marqueur 20 selon l'axe 44 principal est représentée en ordonnée ; le temps est représenté en abscisse. La période d'enregistrement comporte plusieurs cycles 41 respiratoires du patient.

La figure 4 représente également, en trait discontinu, un modèle prédictif 43 du mouvement du marqueur 20 élaboré à partir du mouvement 40 suivi par le marqueur 20 pendant la période d'enregistrement 42. Dans l'exemple considéré et illustré à la figure 4, le modèle prédictif 43 est une simple répétition du mouvement 40 du marqueur 20 enregistré pendant la période d'enregistrement 42. Il convient cependant de noter que d'autres méthodes peuvent être envisagées pour obtenir un modèle prédictif du mouvement du marqueur. Il est par exemple envisageable de modéliser un cycle respiratoire correspondant à une moyenne des cycles respiratoires observés pendant la période d'enregistrement. Dans des variantes, des algorithmes d'apprentissage automatique peuvent être utilisés pour élaborer le modèle prédictif du mouvement du marqueur à partir du mouvement du marqueur enregistré pendant la période d'enregistrement.

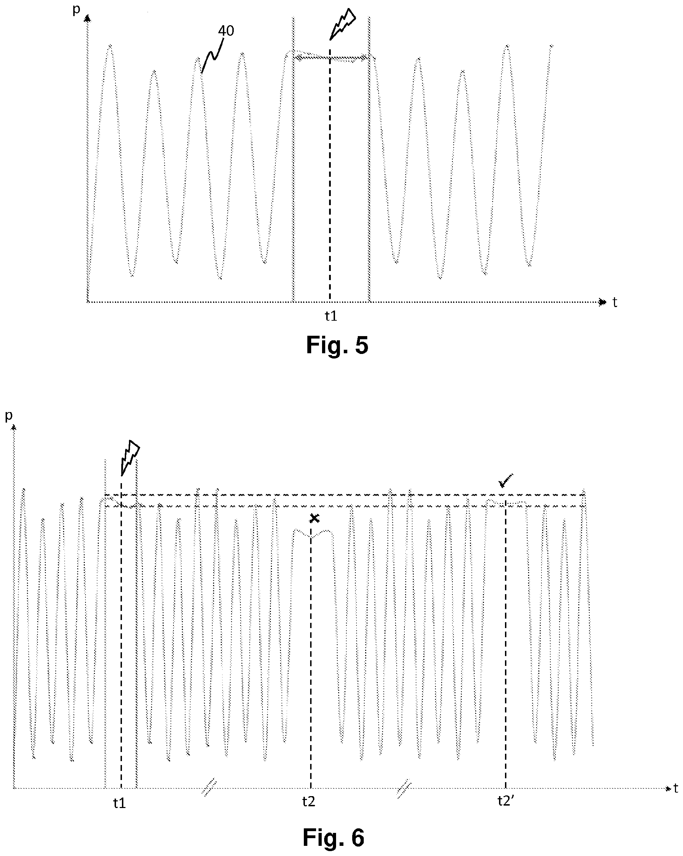

La figure 5 illustre la détermination d'un premier instant t1 appartenant à la période d'enregistrement 42 et correspondant à l'instant où est acquise l'image médicale pré-interventionnelle de l'anatomie d'intérêt du patient. Pour rappel, l'image médicale pré-interventionnelle est acquise par un dispositif d'imagerie médicale qui ne fait pas partie du système de navigation selon l'invention.

Différentes méthodes peuvent être envisagées pour que l'unité de contrôle 13 du système 10 de navigation en réalité augmentée soit capable de déterminer le premier instant t1. Selon un premier exemple, le dispositif d'imagerie médicale peut être connecté avec l'unité de contrôle 13 du système 10 de navigation, par exemple via des moyens de communication filaire ou des moyens de communication sans fil. Dans ce cas le dispositif d'imagerie médicale peut envoyer une indication à l'unité de contrôle 13 à l'instant où l'image médicale pré-interventionnelle est acquise. Selon un deuxième exemple, la position du marqueur 20 au premier instant t1 est enregistrée automatiquement par l'unité de contrôle 13 lorsque le patient est mis en apnée. Dans ce cas, le respirateur ou le dispositif de ventilation est connecté avec l'unité de contrôle 13 du système 10 de navigation, par exemple via des moyens de communications filaires ou des moyens de communication sans fil, et envoie une indication à l'unité de contrôle 13 dès que la respiration du patient est bloquée ou mise en apnée. Selon un troisième exemple, la position du marqueur 20 au premier instant t1 est enregistrée manuellement par l'opérateur lorsque le patient est mis en apnée. Selon un quatrième exemple, la position du marqueur 20 au premier instant t1 est enregistrée automatiquement par l'unité de contrôle 13 dès lors que le marqueur 20 est sensiblement immobile pendant plus de deux secondes (période correspondant par exemple à un plateau du cycle de respiration en fin d'inspiration ou en fin d'expiration). Selon un cinquième exemple, un détecteur de rayons X (par exemple un dosimètre ou un scintillateur) peut être connecté à l'unité de contrôle 13 du système 10 de navigation. Dans ce cas le détecteur de rayons X peut envoyer une indication à l'unité de contrôle 13 à l'instant où l'image médicale pré-interventionnelle est acquise. Pour le deuxième, le troisième et le quatrième exemple, l'image médicale pré-interventionnelle est acquise à un instant où la respiration du patient est bloquée. Pour le premier et le cinquième exemple, il n'est pas indispensable que l'image médicale pré-interventionnelle soit acquise à un instant où la respiration du patient est bloquée.

Sur l'exemple illustré à la figure 5, le premier instant t1 correspond à un instant médian de la durée d'acquisition de l'image médicale. Il est alors possible de déterminer la position prise par le marqueur 20 audit instant t1. Il convient toutefois de noter que le premier instant t1 pourrait avoir une certaine durée (durée d'exposition aux rayons X correspondant à la durée d'acquisition de l'image médicale, voire de plusieurs images médicales consécutives). Dans ce cas, la position prise par le marqueur 20 au premier instant t1 peut être déterminée à partir d'une moyenne des positions prises par le marqueur 20 pendant la durée d'exposition aux rayons X. Il est également envisageable de pondérer cette moyenne par la dose de rayons X reçue à chaque position.

Dans l'exemple illustré à la figure 5, l'image médicale pré-interventionnelle est acquise à un instant où la respiration du patient est bloquée. Rien n'empêche cependant, selon un autre exemple, que l'image médicale pré-interventionnelle soit acquise à un instant où le patient respire librement.

La figure 6 illustre la détermination d'un deuxième instant t2 correspondant à un instant candidat pour l'insertion d'un instrument médical, et la comparaison de la position du marqueur au premier instant t1 (position « cible ») avec la position du marqueur au deuxième instant t2 (position « candidate »). Le deuxième instant t2 est déterminé à partir du modèle prédictif pour que la position candidate corresponde sensiblement à la position cible. Tel qu'illustré sur la figure 6, le mouvement réel du marqueur 20 est suivi de façon continue. Une comparaison de la position candidate du marqueur 20 à l'instant t2 peut alors être effectuée avec la position cible du marqueur 20 à l'instant t1. Dans l'exemple illustré à la figure 6, il se trouve que la position candidate est trop éloignée de la position cible. Un nouveau deuxième instant t2' est alors déterminé à partir du modèle prédictif. La position du marqueur à l'instant t2' est cette fois suffisamment proche de la position cible. Cela signifie que le geste chirurgical (insertion de l'instrument médical) peut avoir lieu à l'instant t2' tel qu'il a été planifié sur l'image médicale pré-interventionnelle.

Pour effectuer la comparaison d'une position candidate avec la position cible, une plage de tolérance est utilisée. Par exemple, on considère que la position candidate est sensiblement identique à la position cible si la position candidate se trouve à ±10% de la position cible. Préférentiellement, une plage de tolérance de ±5% de la position cible est utilisée.

Le résultat de la comparaison est affiché sur le dispositif d'affichage 12 pour indiquer au praticien 31 s'il est opportun ou non de procéder à l'insertion de l'instrument à l'instant candidat considéré.

Dans des modes particuliers de réalisation, le système 10 de navigation en réalité augmentée est configuré pour coopérer avec un robot médical utilisé pour assister le praticien dans son exercice du geste chirurgical, ou pour effectuer directement le geste chirurgical de façon autonome. Le résultat de la comparaison est alors envoyé par le système 10 de navigation au robot médical pour activer, le cas échéant, le positionnement d'un bras articulé du robot médical de façon appropriée pour l'exécution du geste chirurgical.

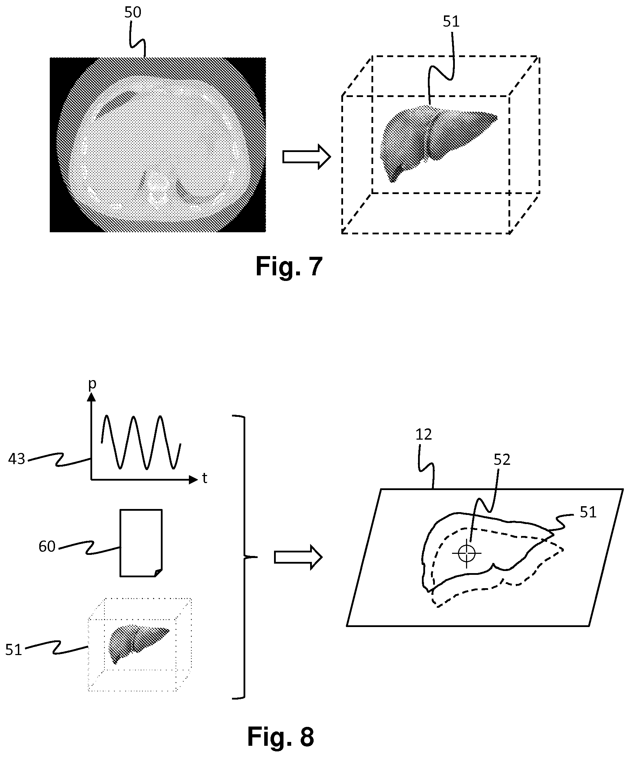

De façon indépendante à la détermination d'un instant opportun pour l'exécution du geste chirurgical, et tel qu'illustré sur les figures 7 et 8, le modèle prédictif 43 du mouvement du marqueur 20 peut également être utilisé pour mettre à jour de façon continue et en temps réel un modèle anatomique 51 en trois dimensions de l'anatomie d'intérêt généré par l'unité de contrôle 13 à partir de l'image médicale pré-interventionnelle 50. Ce modèle anatomique peut alors être affiché sur le dispositif d'affichage 12 sous forme d'un contenu de réalité augmenté superposé aux images réelles.

Pour recevoir l'image médicale pré-interventionnelle acquise par le dispositif d'imagerie médicale, l'unité de contrôle est par exemple connectée au dispositif d'imagerie via des moyens de communication filaire ou des moyens de communication sans fil. Selon un autre exemple, l'unité de contrôle peut être connectée à un périphérique mémoire, par exemple une clé USB (acronyme de « Universal Serial Bus », en français « bus universel en série ») sur laquelle est enregistrée l'image médicale pré-interventionnelle.

Dans l'exemple considéré et illustré aux figures 7 et 8, l'anatomie d'intérêt est le foie du patient, et l'intervention chirurgicale vise à ablater une tumeur présente dans ladite anatomie d'intérêt. L'image médicale pré-interventionnelle 50 est acquise par tomodensitométrie assistée par ordinateur (CT-scan). L'instant auquel l'image médicale est acquise, ainsi que la position correspondante du marqueur 20 à cet instant, sont déterminés par l'unité de contrôle 13 du système 10 de navigation.

L'unité de contrôle 13 est configurée afficher le modèle anatomique 51 sur le dispositif d'affichage 12 sous forme d'un contenu de réalité augmenté superposé aux images réelles. Toutefois, les mouvements engendrés par la respiration du patient entraînent un mouvement de l'anatomie d'intérêt. Tel qu'illustré sur la figure 8, l'unité de contrôle est configurée pour mettre à jour de façon continue et en temps réel la position du modèle anatomique 51 en fonction du modèle prédictif 43 du mouvement du marqueur et en fonction d'un modèle biomécanique 60 de structures anatomiques du corps humain. Ainsi, le modèle anatomique 51 affiché sur le dispositif d'affichage 12 en superposition par rapport aux images réelles est en mouvement de façon synchronisée avec la respiration du patient.

La position du modèle anatomique 51 au moment où l'image médicale pré-interventionnelle est acquise correspond à une position de référence du modèle anatomique 51 par rapport à une position particulière du marqueur 20. Grâce au modèle prédictif 43 du mouvement du marqueur 20, il est possible d'anticiper la position que va prendre le marqueur 20 au cours du temps. Grâce au modèle biomécanique 60, il est possible d'ajuster la position du modèle anatomique 51 en fonction de la position prise par le marqueur 20 au cours du temps.

Le modèle biomécanique 60 inclut de préférence les principales structures anatomiques de la zone thoraco-abdomino-pelvienne telles que les parois thoracique et abdominale, les muscles, les tendons, les os et articulations, les organes, le réseau vasculaire, etc., ainsi que leurs modèles de déformation et leurs interactions mécaniques. Le modèle biomécanique 60 prend en compte également, de préférence, les effets de la gravité en fonction de la position du patient 30. De tels modèles biomécaniques sont connus dans la littérature scientifique, voir par exemple les publications suivantes :
- « SOFA : A Multi-Model Framework for Interactive Physical Simulation », F. Faure et al., Soft Tissue Biomechanical Modeling for Computer Assisted Surgery - Studies in Mechanobiology, Tissue Engineering and Biomaterials, Volume 11, Springer,

- « A Personalized Biomechanical Model for Respiratory Motion Prediction », B. Fuerst et al., International Conference on Medical Image Computing and Computer Assisted Intervention, 2012,
- « Patient-Specific Biomechanical Model as Whole-Body CT Image Registration Tool », Mao Li et al., Medical Image Analysis, 2015, May, pages 22-34.

Il est à noter que le modèle biomécanique du corps humain n'est pas nécessairement spécifique au patient considéré, et peut être un modèle biomécanique d'un patient générique, par exemple de même sexe, taille, corpulence, etc. que le patient considéré sur lequel doit être réalisé le geste chirurgical.

L'unité de contrôle 13 intègre des algorithmes de mise en correspondance du modèle biomécanique 60 avec la position du marqueur 20 situé au niveau de la peau du patient 30, à proximité de l'anatomie d'intérêt. Par exemple, un algorithme permet de propager les mouvements de la surface de la peau au volume interne et de calculer correctement la position des structures anatomiques internes.

L'unité de contrôle 13 peut également être configurée pour afficher sur le modèle anatomique 51, sous forme d'un contenu de réalité augmenté superposé aux images réelles, une trajectoire destinée à être suivie par un instrument médical. La trajectoire est alors mise à jour en temps réel en fonction de la position du modèle anatomique. Sur l'exemple illustré à la figure 8, la trajectoire comporte un point cible 52 de l'anatomie d'intérêt. Ce point cible 52 correspond par exemple au centre de la tumeur à traiter. La trajectoire peut également comporter un point d'entrée de l'instrument médical à la surface de l'anatomie d'intérêt. La trajectoire destinée à être suivie par l'instrument médical est généralement définie à partir de l'image médicale pré-interventionnelle 50.

Toutefois, il est également envisageable de définir cette trajectoire sur une image pré-opératoire, plusieurs jours voire plusieurs mois avant l'intervention. Dans ce cas, l'unité de contrôle 13 est configurée pour effectuer un recalage de l'image pré-opératoire avec l'image pré-interventionnelle. Dans ce but, l'unité de contrôle peut mettre en œuvre des algorithmes conventionnels de recalage et/ou de fusion d'images médicales.

L'unité de contrôle reçoit par exemple l'image médicale pré-opératoire via des moyens de communication filaire ou des moyens de communication sans fil. Selon un autre exemple, l'unité de contrôle peut être connectée à un périphérique mémoire (par exemple une clé USB) sur laquelle est enregistrée l'image médicale pré-opératoire.

Certains éléments peuvent être segmentés sur le modèle anatomique 51, comme par exemple une région à traiter (la tumeur à ablater), une région d'ablation estimée en fonction des paramètres de traitement, ou des marges d'ablation de la région à traiter (comparaison de la région à traiter avec la région d'ablation estimée). Ces éléments peuvent également être affichés sous la forme d'un contenu de réalité augmenté. Leurs positions sont alors mises à jour en temps réel en fonction de la position du modèle anatomique.

Dans des modes particuliers de réalisation, le système 10 de navigation en réalité augmentée comporte un dispositif d'interaction permettant au praticien 31 d'interagir avec ledit système 10. Le dispositif d'interaction prend par exemple la forme d'un gant de réalité virtuelle permettant de capturer la position exacte et la pression exercée par les doigts et fournissant un retour haptique permettant à l'utilisateur de ressentir des objets virtuels. Selon un autre exemple, le dispositif d'interaction peut prendre la forme d'un stylet permettant de pointer un objet virtuel ou un endroit particulier du modèle anatomique. Le dispositif d'interaction peut notamment permettre au praticien de définir ou de modifier la trajectoire destinée à être suivie par l'instrument médical. Le dispositif d'interaction peut également permettre d'interagir avec des objets virtuels tels que des menus de configuration, des boutons, des listes de choix multiples, etc., pour définir des valeurs particulières de paramètres pour le traitement à effectuer (par exemple le type de traitement à réaliser, la durée du traitement, la puissance ou la dose à utiliser pour le traitement, etc.). Alternativement, la trajectoire que doit suivre l'instrument médical et/ou les paramètres de traitement sont déterminés automatiquement par des algorithmes d'apprentissage automatique. Le praticien peut alors valider ou modifier la trajectoire et/ou les paramètres de traitement proposés.

La position de certains contenus de réalité augmentée affichés sur le dispositif d'affichage 12 peut être adaptée en fonction de la position de la tête du praticien. Par exemple, lorsque le praticien penche la tête vers la gauche, certains objets virtuels de configuration affichés en réalité augmentée (notamment des objets virtuels prenant la forme de texte) peuvent subir une rotation dans le sens trigonométrique de telle sorte que ces éléments apparaissent au praticien comme s'il ne penchait pas la tête. La position de la tête du praticien peut notamment être déterminée à l'aide d'un capteur de mouvement de la tête du praticien, ou bien directement à partir de la position du marqueur 20 sur les images réelles.

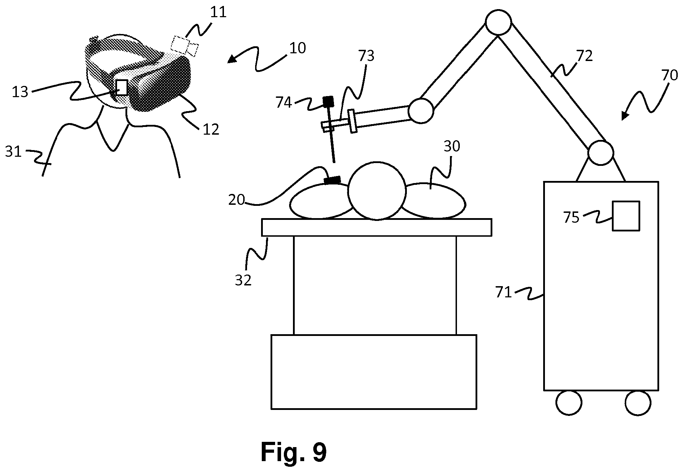

Tel qu'illustré sur la figure 9, le système 10 de navigation en réalité augmentée peut coopérer avec un robot médical 70 utilisé pour assister le praticien 31 pendant l'intervention chirurgicale. Le robot médical comporte une base mobile 71, un bras articulé 72, et une unité de contrôle 75. Une extrémité du bras articulé 72 est fixée à la base mobile 71 et l'autre extrémité présente un guide outil 73 destiné à maintenir un instrument médical 74. L'unité de contrôle 75 du robot médical est configurée pour déterminer une configuration du bras articulé qui permette d'effectuer un geste chirurgical avec l'instrument médical selon une trajectoire prédéterminée. La configuration du bras articulé est déterminée en fonction d'une information relative à la position du marqueur 20 transmise par le système 10 de navigation. Le système 10 de navigation en réalité augmentée et le robot médical 70 comportent par exemple des moyens de communication pour échanger des messages. Ces moyens de communication peuvent être de type filaire ou sans fil.

Le guide outil 73 est par exemple constitué de deux pinces, entrainées par un actionneur linéaire par l'intermédiaire de deux bielles afin de maintenir ou de libérer l'instrument médical 74. L'actionneur linéaire peut être réversible (le guide outil 73 peut alors être ouvert manuellement ou automatiquement sur commande de l'unité de contrôle 75) ou non réversible (le guide outil 73 ne peut être ouvert que de façon automatique sur commande de l'unité de contrôle 75). Avantageusement, le guide outil 73 permet de guider des instruments médicaux 74 de différents diamètres tout en conservant la position de l'axe de guidage de l'instrument médical 74 et de libérer latéralement l'instrument médical 74 à tout moment de l'intervention. Par exemple, un tel guide outil 73 permet de guider des instruments dont le diamètre externe est compris entre 11 et 21 gauges (G) (la gauge est une unité de mesure couramment utilisée pour définir le diamètre externe d'un instrument médical tel qu'une aiguille, une sonde ou un cathéter ; 11 gauges correspondent à un diamètre de 2,946 mm ; 21 gauges correspondent à un diamètre de 0,812 mm).

Le geste médical peut être effectué par le praticien 31 (dans ce cas le bras articulé 72 du robot médical 70 sert principalement à guider l'instrument médical 74 pour assister le praticien dans son exécution du geste médical) ou directement par le robot médical 70.

Dans l'exemple considéré, l'information relative à la position du marqueur 20 correspond à un modèle prédictif représentatif de la position du modèle anatomique 51 au cours des cycles respiratoires du patient. L'unité de contrôle 75 du robot médical 70 peut alors ajuster de façon continue et en temps réel la configuration du bras articulé 72 en fonction dudit modèle prédictif représentatif de la position du modèle anatomique. Ainsi, le bras articulé 72 du robot médical 70 reste continuellement dans une configuration qui permet de réaliser le geste chirurgical planifié.

Dans des modes particuliers de réalisation, et tel qu'illustré sur la figure 9, le bras articulé 72 comporte au moins six degrés de liberté, de sorte que plusieurs configurations candidates 81, 82 différentes du bras articulé permettent d'effectuer le geste chirurgical avec l'instrument médical 74 selon la trajectoire planifiée. L'unité de contrôle 13 du système 10 de navigation en réalité augmentée est configurée pour afficher sur le dispositif d'affichage 12, sous forme d'un contenu de réalité augmenté superposé aux images réelles, lesdites configurations candidates 81, 82. Le praticien peut alors sélectionner l'une de ces configurations candidates à l'aide d'un dispositif d'interaction (par exemple un gant ou un stylet de réalité virtuelle). L'unité de contrôle 13 du système 10 de navigation peut alors communiquer la configuration sélectionnée à l'unité de contrôle 75 du robot médical 70.

Il peut arriver que la configuration sélectionnée du bras articulé ne convienne pas parce que la corpulence du patient est trop importante. Aussi, au cours de l'intervention, il est possible que le patient 30 effectue un mouvement brusque de manière inattendue. Dans de tels cas, il convient de s'assurer que le patient ne soit pas blessé par un contact indésiré entre l'enveloppe corporelle du patient et l'instrument médical ou le bras articulé.

Dans ce but, dans des modes particuliers de réalisation, l'unité de contrôle 13 du système 10 de navigation en réalité augmentée est configurée pour modéliser une enveloppe externe du corps du patient 30. L'enveloppe externe du corps du patient peut être générée à partir de l'image médicale pré-interventionnelle 50 ou à partir du modèle anatomique 51, et éventuellement à l'aide de données morphologiques du patient. Alternativement ou en complément, l'enveloppe externe du corps du patient peut être déterminée sur les images réelles acquises par la caméra 11 du système 10 de navigation (via des algorithmes de détection de contour par exemple). L'unité de contrôle 13 du système 10 de navigation est également configurée pour déterminer la position du bras articulé 72 ou du guide outil 73 du robot médical 70 et pour détecter une situation de risque de collision lorsque la distance entre le bras articulé 72 ou le guide outil 73 et l'enveloppe externe du corps du patient 30 est inférieure à un seuil prédéterminé. L'unité de contrôle 75 peut alors être configurée pour interrompre le mouvement du bras articulé 72 dès que la distance entre le bras articulé 72 ou le guide outil 73 et l'enveloppe externe du patient est insuffisante,

Alternativement ou en complément, dans des modes particuliers de réalisation, l'unité de contrôle 13 du système 10 de navigation en réalité augmentée est configurée pour mesurer une déviation de la position du marqueur 20 par rapport au modèle prédictif 43 du mouvement du marqueur 20 (la déviation à un instant donné correspond par exemple à la distance entre la position réelle du marqueur audit instant et la position du marqueur dans le modèle prédictif 43 pour un instant correspondant du cycle de respiration du patient). L'unité de contrôle 13 du système 10 de navigation en réalité augmentée est alors configurée pour détecter une situation de risque de blessure par l'instrument médical 74 lorsque la déviation ainsi mesurée est supérieure à un seuil prédéterminé. Des mesures peuvent alors être prises pour éviter de blesser le patient lorsqu'une telle situation est détectée. Lorsque la situation de risque de blessure est détectée, l'unité de contrôle 13 du système 10 de navigation en réalité augmentée transmet instantanément cette information à l'unité de contrôle 75 du robot médical 70. L'unité de contrôle 75 peut alors être configurée pour effectuer des actions particulières lorsque la situation de risque de blessure est détectée, comme par exemple commander l'actionneur du guide outil 73 pour libérer instantanément l'instrument médical 74.

Lorsque l'instrument médical 74 est inséré dans le corps du patient 30 selon la trajectoire prédéterminée, l'unité de contrôle 13 du système 10 de navigation en réalité augmentée peut être configurée pour afficher sur le dispositif d'affichage 12, sous forme d'un contenu de réalité augmenté superposé aux images réelles, une partie de l'instrument médical 74 insérée dans le corps du patient 30. Il est également envisageable de calculer et d'afficher la distance entre l'instrument médical 74 et un point cible 52 de l'anatomie d'intérêt. Il est aussi possible de détecter un instant où l'instrument médical 74 a atteint ledit point cible 52 et d'afficher une indication signalant cela au praticien.

La description ci-avant illustre clairement que, par ses différentes caractéristiques et leurs avantages, la présente invention atteint les objectifs fixés, à savoir fournir une solution pour assister un praticien lors d'une intervention chirurgicale mini-invasive en prenant en compte de manière fiable les mouvements liés à la respiration du patient ou à des déplacements involontaires du patient au cours de l'intervention chirurgicale.

## Revendications

1. Système (10) de navigation en réalité augmentée pour assister un praticien (31) lors d'une intervention chirurgicale sur une anatomie d'intérêt d'un patient (30), ledit système (10) comprenant:
- une caméra (11) pour acquérir des images réelles, ladite caméra (11) étant destinée à être portée par le praticien (31) au niveau de la tête,
- un dispositif d'affichage (12) pour afficher en temps réel les images réelles acquises par la caméra (11) ainsi qu'un contenu de réalité augmentée superposé auxdites images réelles,
- une unité de contrôle (13) connectée à la caméra (11) et au dispositif d'affichage (12),
ladite unité de contrôle (13) est configurée pour:
- détecter sur les images réelles la position et l'orientation d'un marqueur (20) placé sur le patient (30) à proximité de ladite anatomie d'intérêt,
- enregistrer le mouvement (40) suivi par le marqueur (20) pendant une période d'enregistrement (42) correspondant à un ou plusieurs cycles respiratoires (41) du patient (30),
- recevoir une information qu'une image médicale pré-interventionnelle de l'anatomie d'intérêt du patient a été acquise par un dispositif d'imagerie médicale à un premier instant (t1) appartenant à ladite période d'enregistrement (42), et déterminer la position et l'orientation du marqueur (20) audit premier instant (t1),
ledit système (10) de navigation en réalité augmentée étant **caractérisé en ce que** ladite unité de contrôle (13) est configurée pour:
- élaborer un modèle prédictif (43) du mouvement du marqueur (20) à partir du mouvement (40) suivi par le marqueur pendant la période d'enregistrement (42).

2. Système (10) selon la revendication 1 dans lequel l'unité de contrôle (13) est en outre configurée pour :
- déterminer à partir du modèle prédictif (43) un deuxième instant (t2) correspondant à un instant candidat d'insertion d'un instrument médical dans l'anatomie d'intérêt du patient,
- comparer la position et l'orientation du marqueur (20) au premier instant (t1) avec la position et l'orientation du marqueur (20) au deuxième instant (t2),
- afficher un résultat de la comparaison sur le dispositif d'affichage (12) sous forme d'un contenu de réalité augmenté superposé aux images réelles.

3. Système (10) selon l'une quelconque des revendications 1 ou 2 dans lequel l'unité de contrôle (13) est en outre configurée pour recevoir l'image médicale pré-interventionnelle (50) du dispositif d'imagerie médicale et pour générer à partir de ladite image médicale pré-interventionnelle (50) un modèle anatomique (51) en trois dimensions de l'anatomie d'intérêt du patient (30) et pour afficher ledit modèle anatomique (51) sur le dispositif d'affichage (12) sous forme d'un contenu de réalité augmenté superposé aux images réelles, la position et l'orientation du modèle anatomique (51) superposé aux images réelles étant mise à jour de façon continue et en temps réel en fonction du modèle prédictif (43) du mouvement du marqueur et en fonction d'un modèle biomécanique (60) de structures anatomiques du corps humain.

4. Système (10) selon la revendication 3 dans lequel l'unité de contrôle (13) est en outre configurée pour afficher sur le modèle anatomique (51), sous forme d'un contenu de réalité augmenté superposé aux images réelles, une trajectoire destinée à être suivie par un instrument médical, ladite trajectoire étant mise à jour en temps réel en fonction de la position et l'orientation du modèle anatomique.

5. Système (10) selon la revendication 4 dans lequel la trajectoire destinée à être suivie par l'instrument médical est définie a priori sur une image médicale pré-opératoire, et l'unité de contrôle (13) est configurée pour recevoir ladite image pré-opératoire et pour effectuer un recalage de l'image pré-opératoire avec l'image médicale pré-interventionnelle (50) pour afficher la trajectoire sur le modèle anatomique (51).

6. Système (10) selon la revendication 4 comportant en outre un dispositif d'interaction permettant au praticien (31) de pointer un endroit particulier sur le modèle anatomique (51) et dans lequel la trajectoire destinée à être suivie par l'instrument médical est définie par le praticien à l'aide du dispositif d'interaction.

7. Système (10) selon l'une quelconque des revendications 3 à 6 dans lequel l'unité de contrôle (13) est configurée pour segmenter au moins un élément sur le modèle anatomique (51) parmi les éléments suivants:
- différentes structures anatomiques au sein de l'anatomie d'intérêt,
- une région à traiter au sein de l'anatomie d'intérêt,
- une région d'ablation estimée à partir de paramètres de l'intervention chirurgicale,
- des marges d'ablation d'une région à traiter au sein de l'anatomie d'intérêt déterminées en comparant la région à traiter et la région d'ablation estimée,
et pour afficher ledit élément segmenté sur le modèle anatomique (51) sous forme d'un contenu de réalité augmenté superposé aux images réelles, l'élément segmenté étant mis à jour en temps réel en fonction de la position et l'orientation du modèle anatomique.

8. Système (10) selon l'une quelconque des revendications 1 à 7 dans lequel l'unité de contrôle (13) est configurée pour afficher sur le dispositif d'affichage (12), sous forme d'un contenu de réalité augmenté, au moins un objet virtuel de configuration concernant un paramètre de traitement chirurgical, et le système (10) comporte un dispositif d'interaction permettant au praticien (31) d'interagir avec ledit objet virtuel de configuration afin de sélectionner une valeur particulière pour ledit paramètre.

9. Système (10) selon l'une quelconque des revendications 1 à 8 dans lequel le dispositif d'affichage (12) est un écran d'affichage (12a) intégré dans un casque de réalité augmentée.

10. Système (10) selon l'une quelconque des revendications 1 à 9 dans lequel l'unité de contrôle (13) est configurée pour modifier la position et l'orientation d'un contenu de réalité augmentée en fonction de la position et l'orientation de la tête du praticien (31).

11. Système (10) selon la revendication 10 dans lequel la position et l'orientation de la tête du praticien (31) est déterminée à l'aide d'un capteur de mouvement de la tête du praticien.

12. Ensemble formé par un système (10) de navigation en réalité augmentée selon l'une quelconque des revendications 1 à 11 et un robot médical (70) pour assister un praticien (31) lors d'une intervention chirurgicale sur une anatomie d'intérêt d'un patient (30), le robot médical (70) comportant :
- une base mobile (71),
- un bras articulé (72) dont une extrémité est fixée à la base mobile (71) et l'autre extrémité présente un guide outil (73) destiné à maintenir un instrument médical (74),
- une unité de contrôle (75) configurée pour déterminer une configuration du bras articulé (72) qui permette d'effectuer un geste chirurgical avec l'instrument médical (74) selon une trajectoire prédéterminée, la configuration du bras articulé étant déterminée en fonction d'une information relative à la position et l'orientation du marqueur (20) transmise par le système (10) de navigation.

13. Ensemble selon la revendication 12 en combinaison avec la revendication 2, dans lequel l'information relative à la position et l'orientation du marqueur (20) est une indication que la différence entre la position et l'orientation du marqueur (20) au premier instant (t1) et la position et l'orientation du marqueur (20) au deuxième instant (t2) est inférieure à un seuil prédéterminé.

14. Ensemble selon la revendication 12 en combinaison avec la revendication 3, dans lequel l'information relative à la position et l'orientation du marqueur (20) correspond à un modèle prédictif représentatif de la position et l'orientation du modèle anatomique (51) au cours des cycles respiratoires du patient, et l'unité de contrôle (75) du robot médical (70) est configurée pour ajuster de façon continue et en temps réel la configuration du bras articulé (72) en fonction dudit modèle prédictif représentatif de la position et l'orientation du modèle anatomique.

15. Ensemble selon l'une quelconque des revendications 12 à 14 dans lequel l'unité de contrôle (13) du système (10) de navigation en réalité augmentée est configurée pour:
- modéliser une enveloppe externe du corps du patient (30),
- déterminer la position et l'orientation du bras articulé (72) ou du guide outil (73) du robot médical (70),
- détecter une situation de risque de collision lorsque la distance entre le bras articulé (72) ou le guide outil (73) du robot médical (70) et l'enveloppe externe du patient (30) est inférieure à un seuil prédéterminé.

16. Ensemble selon l'une quelconque des revendications 12 à 15 dans lequel l'unité de contrôle (13) du système (10) de navigation en réalité augmentée est configurée pour détecter une situation de risque de blessure par l'instrument médical (74) lorsqu'une déviation de la position et l'orientation du marqueur (20) par rapport au modèle prédictif (43) du mouvement du marqueur (20) est supérieure à un seuil prédéterminé.

17. Ensemble selon la revendication 16 dans lequel le guide outil (73) du robot médical (70) comporte un actionneur permettant de libérer instantanément l'instrument médical (74), ledit actionneur étant commandé par l'unité de contrôle (75) du robot médical (70), et l'unité de contrôle (13) du système (10) de navigation en réalité augmentée est configurée pour transmettre à l'unité de contrôle (75) du robot médical (70) une commande pour libérer instantanément l'instrument médical (74) lorsque la situation de risque de blessure est détectée.

18. Ensemble selon l'une quelconque des revendications 12 à 17 dans lequel le bras articulé (72) comporte au moins six degrés de liberté, de sorte que plusieurs configurations candidates (81, 82) différentes du bras articulé (72) permettent d'effectuer le geste chirurgical avec l'instrument médical (74) selon la trajectoire prédéterminée, et l'unité de contrôle (13) du système (10) de navigation en réalité augmentée est configurée pour afficher sur le dispositif d'affichage (12), sous forme d'un contenu de réalité augmenté superposé aux images réelles, lesdites configurations candidates (81, 82), et pour recevoir une indication relative à une sélection, par le praticien (31), d'une configuration particulière parmi les différentes configurations candidates (81, 82).

19. Ensemble selon l'une quelconque des revendications 12 à 18 dans lequel, lorsque l'instrument médical (74) est inséré dans le corps du patient (30) selon la trajectoire prédéterminée, l'unité de contrôle (13) du système (10) de navigation en réalité augmentée est configurée pour afficher sur le dispositif d'affichage (12), sous forme d'un contenu de réalité augmenté superposé aux images réelles, une partie de l'instrument médical (74) insérée dans le corps du patient (30).

20. Ensemble selon la revendication 19 dans lequel l'unité de contrôle (13) du système (10) de navigation en réalité augmentée est configurée pour calculer et afficher sur le dispositif d'affichage (12), sous forme d'un contenu de réalité augmenté superposé aux images réelles, la distance entre l'instrument médical (74) et un point cible (52) de l'anatomie d'intérêt et/ou pour détecter un instant où l'instrument médical (74) a atteint ledit point cible (52).

## Patentansprüche

1. Augmented-Reality-Navigationssystem (10) zur Unterstützung eines Arztes (31) bei einem chirurgischen Eingriff an einer Anatomie von Interesse eines Patienten (30), wobei das System (10) Folgendes umfasst:
- eine Kamera (11) zum Erfassen realer Bilder, wobei die Kamera (11) dazu bestimmt ist, vom Arzt (31) am Kopf getragen zu werden,
- eine Anzeigevorrichtung (12) zum Anzeigen der von der Kamera (11) erfassten realen Bilder in Echtzeit sowie eines Augmented-Reality-Inhalts, der den realen Bildern überlagert ist,
- eine Steuereinheit (13), die mit der Kamera (11) und der Anzeigevorrichtung (12) verbunden ist, wobei die Steuereinheit (13) zu Folgendem konfiguriert ist:
- Erkennen der Position und der Ausrichtung eines am Patienten (30) in der Nähe der Anatomie von Interesse angebrachten Markers (20) auf den realen Bildern,
- Aufzeichnen der Bewegung (40), der der Marker (20) folgt, während eines Aufzeichnungszeitraums (42), der einem oder mehreren Atemzyklen (41) des Patienten (30) entspricht,
- Empfangen einer Information, dass ein präoperatives medizinisches Bild der Anatomie von Interesse des Patienten durch eine medizinische Bildgebungsvorrichtung zu einem ersten Zeitpunkt (t1) erfasst wurde, der zum Aufzeichnungszeitraum (42) gehört, und Bestimmen der Position und der Ausrichtung des Markers (20) zu dem ersten Zeitpunkt (t1), wobei das Augmented-Reality-Navigationssystem (10) **dadurch gekennzeichnet ist, dass** die Steuereinheit (13) zu Folgendem konfiguriert ist:
- Erstellen eines Vorhersagemodells (43) der Bewegung des Markers (20) aus der Bewegung (40), die vom Marker während des Aufzeichnungszeitraums (42) verfolgt wird.

2. System (10) nach Anspruch 1, wobei die Steuereinheit (13) ferner zu Folgendem konfiguriert ist:
- Bestimmen, aus dem Vorhersagemodell (43), eines zweiten Zeitpunkts (t2), der einem Kandidatenzeitpunkt für das Einführen eines medizinischen Instruments in die Anatomie von Interesse des Patienten entspricht,
- Vergleichen der Position und der Ausrichtung des Markers (20) zu dem ersten Zeitpunkt (t1) mit der Position und der Ausrichtung des Markers (20) zu dem zweiten Zeitpunkt (t2),
- Anzeigen eines Ergebnisses des Vergleichs in Form eines Augmented-Reality-Inhalts, der den realen Bildern überlagert ist, auf der Anzeigevorrichtung (12).

3. System (10) nach einem der Ansprüche 1 oder 2, wobei die Steuereinheit (13) ferner so konfiguriert ist, dass sie das präoperative medizinische Bild (50) der medizinischen Bildgebungsvorrichtung empfängt und aus dem präoperativen medizinischen Bild (50) ein dreidimensionales anatomisches Modell (51) der Anatomie von Interesse des Patienten (30) erzeugt und das anatomische Modell (51) auf der Anzeigevorrichtung (12) in Form eines Augmented-Reality-Inhalts anzeigt, der den realen Bildern überlagert ist, wobei die Position und die Ausrichtung des anatomischen Modells (51), das den realen Bildern überlagert ist, kontinuierlich und in Echtzeit entsprechend dem Vorhersagemodell (43) der Bewegung des Markers und entsprechend eines biomechanischen Modells (60) der anatomischen Strukturen des menschlichen Körpers aktualisiert wird.

4. System (10) nach Anspruch 3, wobei die Steuereinheit (13) ferner so konfiguriert ist, dass sie auf dem anatomischen Modell (51) in Form eines Augmented-Reality-Inhalts, der den realen Bildern überlagert ist, einen Pfad anzeigt, der von einem medizinischen Instrument verfolgt werden soll, wobei der Pfad in Echtzeit entsprechend der Position und der Ausrichtung des anatomischen Modells aktualisiert wird.

5. System (10) nach Anspruch 4, wobei der Pfad, der vom medizinischen Instrument verfolgt werden soll, a priori auf einem präoperativen medizinischen Bild definiert ist, und die Steuereinheit (13) so konfiguriert ist, dass sie das präoperative Bild empfängt und eine Bildregistrierung des präoperativen Bildes mit dem präoperativen medizinischen Bild (50) durchführt, um den Pfad auf dem anatomischen Modell (51) anzuzeigen.

6. System (10) nach Anspruch 4, ferner umfassend eine Interaktionsvorrichtung, die es dem Arzt (31) ermöglicht, auf eine bestimmte Stelle des anatomischen Modells (51) zu zeigen, und wobei der Pfad, der vom medizinischen Instrument verfolgt werden soll, vom Arzt mithilfe der Interaktionsvorrichtung definiert wird.

7. System (10) nach einem der Ansprüche 3 bis 6, wobei die Steuereinheit (13) so konfiguriert ist, dass sie mindestens ein Element auf dem anatomischen Modell (51) von den folgenden Elementen segmentiert:
- verschiedene anatomische Strukturen innerhalb der Anatomie von Interesse,
- eine zu behandelnde Region innerhalb der Anatomie von Interesse,
- eine Ablationsregion, die anhand von Parametern des chirurgischen Eingriffs geschätzt wird,
- die Ablationsränder einer zu behandelnden Region innerhalb der Anatomie von Interesse, die durch Vergleichen der zu behandelnden Region und der geschätzten Ablationsregion bestimmt werden,
und das segmentierte Element auf dem anatomischen Modell (51) in Form eines Augmented-Reality-Inhalts, der den realen Bildern überlagert ist, anzeigt, wobei das segmentierte Element in Echtzeit entsprechend der Position und der Ausrichtung des anatomischen Modells aktualisiert wird.

8. System (10) nach einem der Ansprüche 1 bis 7, wobei die Steuereinheit (13) so konfiguriert ist, dass sie in Form eines Augmented-Reality-Inhalts mindestens ein virtuelles Konfigurationsobjekt bezüglich eines chirurgischen Behandlungsparameters auf der Anzeigevorrichtung (12) anzeigt, und das System (10) eine Interaktionsvorrichtung umfasst, die es dem Arzt (31) ermöglicht, mit dem virtuellen Konfigurationsobjekt zu interagieren, um einen bestimmten Wert für den Parameter auszuwählen.

9. System (10) nach einem der Ansprüche 1 bis 8, wobei die Anzeigevorrichtung (12) ein in ein Augmented-Reality-Headset integrierter Anzeigebildschirm (12a) ist.

10. System (10) nach einem der Ansprüche 1 bis 9, wobei die Steuereinheit (13) so konfiguriert ist, dass sie die Position und die Ausrichtung eines Augmented-Reality-Inhalts entsprechend der Position und der Ausrichtung des Kopfes des Arztes (31) ändert.

11. System (10) nach Anspruch 10, wobei die Position und die Ausrichtung des Kopfes des Arztes (31) mithilfe eines Bewegungssensors für den Kopf des Arztes bestimmt wird.

12. Baugruppe, die aus einem Augmented-Reality-Navigationssystem (10) nach einem der Ansprüche 1 bis 11 und einem medizinischen Roboter (70) besteht, um einen Arzt (31) bei einem chirurgischen Eingriff an einer Anatomie von Interesse eines Patienten (30) zu unterstützen, wobei der medizinische Roboter (70) Folgendes umfasst:
- eine bewegliche Basis (71),
- einen Gelenkarm (72), dessen eines Ende an der beweglichen Basis (71) befestigt ist und dessen anderes Ende eine Werkzeugführung (73) aufweist, die zum Halten eines medizinischen Instruments (74) bestimmt ist,
- eine Steuereinheit (75), die so konfiguriert ist, dass sie eine Konfiguration des Gelenkarms (72) bestimmt, die es ermöglicht, eine chirurgische Geste mit dem medizinischen Instrument (74) entlang eines vorbestimmten Pfads durchzuführen, wobei die Konfiguration des Gelenkarms entsprechend einer Information über die Position und die Ausrichtung des Markers (20) bestimmt wird, die vom Navigationssystem (10) übertragen wird.

13. Baugruppe nach Anspruch 12 in Kombination mit Anspruch 2, wobei die Information über die Position und die Ausrichtung des Markers (20) eine Angabe darüber ist, dass der Unterschied zwischen der Position und der Ausrichtung des Markers (20) zu dem ersten Zeitpunkt (t1) und der Position und der Ausrichtung des Markers (20) zu dem zweiten Zeitpunkt (t2) kleiner als ein vorbestimmter Schwellenwert ist.

14. Baugruppe nach Anspruch 12 in Kombination mit Anspruch 3, wobei die Information über die Position und die Ausrichtung des Markers (20) einem repräsentativen Vorhersagemodell der Position und der Ausrichtung des anatomischen Modells (51) während der Atemzyklen des Patienten entspricht, und die Steuereinheit (75) des medizinischen Roboters (70) so konfiguriert ist, dass sie die Konfiguration des Gelenkarms (72) kontinuierlich und in Echtzeit entsprechend des Vorhersagemodells anpasst, das repräsentativ für die Position und Ausrichtung des anatomischen Modells ist.

15. Baugruppe nach einem der Ansprüche 12 bis 14, wobei die Steuereinheit (13) des Augmented-Reality-Navigationssystems (10) zu Folgendem konfiguriert ist:
- Modellieren einer äußeren Hülle des Körpers des Patienten (30),
- Bestimmen der Position und der Ausrichtung des Gelenkarms (72) oder der Werkzeugführung (73) des medizinischen Roboters (70),
- Erkennen einer kollisionsgefährdeten Situation, wenn der Abstand zwischen dem Gelenkarm (72) oder der Werkzeugführung (73) des medizinischen Roboters (70) und der äußeren Hülle des Patienten (30) kleiner als ein vorbestimmter Schwellenwert ist.

16. Baugruppe nach einem der Ansprüche 12 bis 15, wobei die Steuereinheit (13) des Augmented-Reality-Navigationssystems (10) so konfiguriert ist, dass sie eine Situation mit Verletzungsrisiko durch das medizinische Instrument (74) erkennt, wenn eine Abweichung der Position und der Ausrichtung des Markers (20) in Bezug auf das Vorhersagemodell (43) der Bewegung des Markers (20) größer als ein vorbestimmter Schwellenwert ist.

17. Baugruppe nach Anspruch 16, wobei die Werkzeugführung (73) des medizinischen Roboters (70) einen Aktuator zum sofortigen Freigeben des medizinischen Instruments (74) umfasst, wobei der Aktuator von der Steuereinheit (75) des medizinischen Roboters (70) gesteuert wird, und die Steuereinheit (13) des Augmented-Reality-Navigationssystems (10) so konfiguriert ist, dass sie an die Steuereinheit (75) des medizinischen Roboters (70) einen Befehl zum sofortigen Freigeben des medizinischen Instruments (74) überträgt, wenn die Situation mit Verletzungsrisiko erkannt wird.

18. Baugruppe nach einem der Ansprüche 12 bis 17, wobei der Gelenkarm (72) mindestens sechs Freiheitsgrade aufweist, so dass mehrere verschiedene Kandidatenkonfigurationen (81, 82) des Gelenkarms (72) es ermöglichen, die chirurgische Geste mit dem medizinischen Instrument (74) entlang des vorbestimmten Pfads durchzuführen, und die Steuereinheit (13) des Augmented-Reality-Navigationssystems (10) so konfiguriert ist, dass sie auf der Anzeigevorrichtung (12) die Kandidatenkonfigurationen (81, 82) in Form eines Augmented-Reality-Inhalts, der den realen Bildern überlagert ist, anzeigt und eine Angabe bezüglich einer Auswahl einer bestimmten Konfiguration aus den verschiedenen Kandidatenkonfigurationen (81, 82) durch den Arzt (31) empfängt.

19. Baugruppe nach einem der Ansprüche 12 bis 18, wobei, wenn das medizinische Instrument (74) entlang des vorbestimmten Pfads in den Körper des Patienten (30) eingeführt wird, die Steuereinheit (13) des Augmented-Reality-Navigationssystems (10) so konfiguriert ist, dass sie auf der Anzeigevorrichtung (12) einen Teil des in den Körper des Patienten (30) eingeführten medizinischen Instruments (74) in Form eines Augmented-Reality-Inhalts, der den realen Bildern überlagert ist, anzeigt.

20. Baugruppe nach Anspruch 19, wobei die Steuereinheit (13) des Augmented-Reality-Navigationssystems (10) so konfiguriert ist, dass sie den Abstand zwischen dem medizinischen Instrument (74) und einem Zielpunkt (52) der Anatomie von Interesse berechnet und diesen in Form eines Augmented-Reality-Inhalts, der den realen Bildern überlagert ist, auf der Anzeigevorrichtung (12) anzeigt und/oder einen Zeitpunkt erkennt, zu dem das medizinische Instrument (74) den Zielpunkt (52) erreicht hat.

## Claims

1. An augmented-reality navigation system (10) for assisting a practitioner (31) during a surgical procedure on an anatomy of interest of a patient (30), said system (10) comprising:
- a camera (11) for acquiring real images, said camera (11) being intended to be worn by the practitioner (31) on their head,
- a display device (12) for displaying, in real time, the real images acquired by the camera (11) as well as augmented-reality content overlaid on said real images,
- a control unit (13) connected to the camera (11) and to the display device (12), said control unit (13) is configured to:
- detect, on the real images, the position and orientation of a marker (20) placed on the patient (30) in proximity to said anatomy of interest,
- record the movement (40) followed by the marker (20) during a recording period (42) corresponding to one or more respiratory cycles (41) of the patient (30),
- receive information that a pre-procedure medical image of the anatomy of interest of the patient has been acquired by a medical imaging device at a first time point (t1) belonging to said recording period (42), and determine the position and orientation of the marker (20) at said first time point (t1), said augmented-reality navigation system (10) being **characterized in that** said control unit (13) is configured to:
- generate a predictive model (43) of the movement of the marker (20) on the basis of the movement (40) followed by the marker during the recording period (42).

2. The system (10) according to claim 1, wherein the control unit (13) is further configured to:
- determine, on the basis of the predictive model (43), a second time point (t2) corresponding to a candidate time point of insertion of a medical instrument into the anatomy of interest of the patient,
- compare the position and orientation of the marker (20) at the first time point (t1) with the position and orientation of the marker (20) at the second time point (t2),
- display a result of the comparison on the display device (12) in the form of augmented-reality content overlaid on the real images.

3. The system (10) according to any one of claims 1 or 2, wherein the control unit (13) is further configured to receive the pre-procedure medical image (50) from the medical imaging device and to generate, on the basis of said pre-procedure medical image (50), a three-dimensional anatomical model (51) of the anatomy of interest of the patient (30) and to display said anatomical model (51) on the display device (12) in the form of augmented-reality content overlaid on the real images, the position and orientation of the anatomical model (51) overlaid on the real images being updated continuously in real time according to the predictive model (43) of the movement of the marker and according to a biomechanical model (60) of anatomical structures of the human body.

4. The system (10) according to claim 3, wherein the control unit (13) is further configured to display on the anatomical model (51), in the form of augmented-reality content overlaid on the real images, a trajectory intended to be followed by a medical instrument, said trajectory being updated in real time according to the position and orientation of the anatomical model.

5. The system (10) according to claim 4, wherein the trajectory intended to be followed by the medical instrument is defined in principle on a pre-operative medical image, and the control unit (13) is configured to receive said pre-operative image and to perform a realignment of the pre-operative image with the pre-procedure medical image (50) to display the trajectory on the anatomical model (51).

6. The system (10) according to claim 4 further comprising an interaction device allowing the practitioner (31) to point toward a particular location on the anatomical model (51) and wherein the trajectory intended to be followed by the medical instrument is defined by the practitioner using the interaction device.

7. The system (10) according to any one of claims 3 to 6, wherein the control unit (13) is configured to segment at least one element on the anatomical model (51) from the following elements:
- different anatomical structures within the anatomy of interest,
- a region to be treated within the anatomy of interest,
- an ablation region estimated on the basis of parameters of the surgical procedure,
- ablation margins of a region to be treated within the anatomy of interest determined by comparing the region to be treated and the estimated ablation region,
and to display said segmented element on the anatomical model (51) in the form of augmented-reality content overlaid on the real images, the segmented element being updated in real time according to the position and orientation of the anatomical model.

8. The system (10) according to any one of claims 1 to 7 wherein the control unit (13) is configured to display on the display device (12), in the form of augmented-reality content, at least one virtual configuration object relating to a surgical treatment parameter, and the system (10) comprises an interaction device allowing the practitioner (31) to interact with said virtual configuration object in order to select a particular value for said parameter.

9. The system (10) according to any one of claims 1 to 8, wherein the display device (12) is a display screen (12a) integrated into an augmented-reality headset.

10. The system (10) according to any one of claims 1 to 9, wherein the control unit (13) is configured to modify the position and orientation of augmented-reality content according to the position and orientation of the head of the practitioner (31).

11. The system (10) according to claim 10, wherein the position and orientation of the head of the practitioner (31) is determined using a sensor of movement of the practitioner's head.

12. An assembly formed by an augmented-reality navigation system (10) according to any one of claims 1 to 11 and a medical robot (70) for assisting a practitioner (31) during a surgical procedure on an anatomy of interest of a patient (30), the medical robot (70) comprising:
- a mobile base (71),
- an articulated arm (72) of which one end is attached to the mobile base (71) and the other end has a tool guide (73) intended to hold a medical instrument (74),
- a control unit (75) configured to determine a configuration of the articulated arm (72) which makes it possible to perform a surgical gesture with the medical instrument (74) along a predetermined trajectory, the configuration of the articulated arm being determined according to information relating to the position and orientation of the marker (20) transmitted by the navigation system (10).

13. The assembly according to claim 12 in combination with claim 2, wherein the information relating to the position and orientation of the marker (20) is an indication that the difference between the position and orientation of the marker (20) at the first time point (t1) and the position and orientation of the marker (20) at the second time point (t2) is less than a predetermined threshold.

14. The assembly according to claim 12 in combination with claim 3, wherein the information relating to the position and orientation of the marker (20) corresponds to a representative predictive model of the position and orientation of the anatomical model (51) during the respiratory cycles of the patient, and the control unit (75) of the medical robot (70) is configured to adjust, continuously in real time, the configuration of the articulated arm (72) according to said representative predictive model of the position and orientation of the anatomical model.

15. The assembly according to any one of claims 12 to 14 wherein the control unit (13) of the augmented-reality navigation system (10) is configured to:
- model an outer envelope of the body of the patient (30),
- determine the position and orientation of the articulated arm (72) or the tool guide (73) of the medical robot (70),
- detect a collision risk situation when the distance between the articulated arm (72) or the tool guide (73) of the medical robot (70) and the outer envelope of the patient (30) is less than a predetermined threshold.

16. The assembly according to any one of claims 12 to 15, wherein the control unit (13) of the augmented-reality navigation system (10) is configured to detect a situation of risk of injury by the medical instrument (74) when a deviation of the position and orientation of the marker (20) relative to the predictive model (43) of the movement of the marker (20) is greater than a predetermined threshold.

17. The assembly according to claim 16 wherein the tool guide (73) of the medical robot (70) comprises an actuator for instantly releasing the medical instrument (74), said actuator being controlled by the control unit (75) of the medical robot (70), and the control unit (13) of the augmented-reality navigation system (10) is configured to transmit to the control unit (75) of the medical robot (70) a command to instantly release the medical instrument (74) when the injury risk situation is detected.

18. The assembly according to any one of claims 12 to 17, wherein the articulated arm (72) comprises at least six degrees of freedom, such that several different candidate configurations (81, 82) of the articulated arm (72) make it possible to perform the surgical gesture with the medical instrument (74) along the predetermined trajectory, and the control unit (13) of the augmented-reality navigation system (10) is configured to display on the display device (12), in the form of augmented-reality content overlaid on the real images, said candidate configurations (81, 82), and to receive an indication relating to a selection, by the practitioner (31), of a particular configuration from the different candidate configurations (81, 82).

19. The assembly according to any one of claims 12 to 18, wherein, when the medical instrument (74) is inserted into the body of the patient (30) along the predetermined trajectory, the control unit (13) of the augmented-reality navigation system (10) is configured to display on the display device (12), in the form of augmented-reality content overlaid on the real images, a part of the medical instrument (74) inserted into the body of the patient (30).

20. The assembly according to claim 19, wherein the control unit (13) of the augmented-reality navigation system (10) is configured to compute and display on the display device (12), in the form of augmented-reality content overlaid on the real images, the distance between the medical instrument (74) and a target point (52) of the anatomy of interest and/or to detect a time point when the medical instrument (74) has reached said target point (52).
